# EUROPÄISCHE PATENTSCHRIFT

(11) **EP 1 335 190 B1**
(45) Veröffentlichungstag und Bekanntmachung des Hinweises auf die Patenterteilung: **10.09.2014**
(21) Anmeldenummer: 02028281.0
(22) Anmeldetag: 17.12.2002
(51) Int. Cl.: G01D 9/00, A61B 5/00, G01N 33/487

(54) **System mit einem steckbaren Datenübertragungsmodul**
System including plug-in data transfer module
Système comprenant une module enfichable de transmission des données

(30) Priorität: 22.12.2001 DE 10163774
(43) Veröffentlichungstag der Anmeldung: 13.08.2003
(73) Patentinhaber: Roche Diagnostics GmbH, 68305 Mannheim (DE); F. Hoffmann-La Roche AG, 4070 Basel (CH)
(72) Erfinder: Manser, Udo, 69168 Wiesloch (DE); Schaefer, Dieter, 69198 Schriesheim (DE); Schabbach, Michael, 69469 Weinheim (DE); Kuenstler, Peter, 69245 Bammental (DE)

(56) Entgegenhaltungen:
- WO-A-00/45696
- DE-A1- 4 305 058
- DE-A1- 19 703 854

## Beschreibung

Die Erfindung betrifft ein System mit einem steckbaren Datenübertragungsmodul welches elektrische Daten von einem Analysesystem umwandelt und diese umgewandelten Daten galvanisch entkoppelt an eine Datenverarbeitungseinheit weiterleitet.

Die vorliegende Erfindung bezieht sich folglich auf das Gebiet des Datentransfers.

Ein derartiger Datentransfer zwischen einem Analysesystem und einer Datenverarbeitungseinheit erweist sich in neuerer Zeit immer häufiger als nützlich, da oftmals eine Verarbeitung von Analysedaten gewünscht ist, die z. B. ein kontinuierliches Monitoring von Analysedaten erleichtert. Ein kontinuierliches Monitoring von Analysedaten oder z. B. eine statistische Auswertung dieser sind nur einige Möglichkeiten, die ein Analyseverfahren oder dessen Anwendung optimieren.

Obwohl eine Datenverarbeitung vielerlei Vorteile beinhaltet zeigt sich eine Datenverarbeitung häufig als zu umfangreich, um von herkömmlichen Analysegeräten selbst geleistet werden zu können. Zur Datenverarbeitung mittels eines Analysesystems wären folglich zusätzliche bauliche Maßnahmen notwendig, die erhöhte Herstellungskosten erfordern würden. Aus Kostengründen wird deshalb oftmals keine Datenverarbeitungseinheit in das Analysesystem integriert, sondern auf separate Datenverarbeitungseinheiten wie z. B. ein PC zurückgegriffen. Dies hat unter anderem den Vorteil, dass standardmäßig auf die Integration einer Datenverarbeitungseinheit verzichtet werden kann und somit die Herstellungskosten der Analysegeräte für Anwendungsgebiete, in denen eine Datenverarbeitung nicht notwendig ist, nicht unnötig erhöht werden.

Ein Anwendungsgebiet, in dem eine Reduktion der Herstellungskosten von Bedeutung ist, ist beispielsweise der medizinische Bereich. Insbesondere ist dabei das medizinische Anwendungsgebiet gemeint, bei dem die Analysegeräte für einen privaten Gebrauch bestimmt sind. Dies ist dadurch zu begründen, dass für Analysesysteme, die an den Privatmann verkauft werden, oftmals der Verkaufpreis ein wesentliches Kaufkriterium ist.

Trotz der Problematik der kostengünstigen Herstellung der Geräte, will man in Zukunft immer weniger auf die Möglichkeit einer Datenverarbeitung verzichten, da sich besonders im medizinischen Bereich häufig eine Verarbeitung und Auswertung von Analysedaten als sinnvoll erweist.

Eine Überwachung des Gesundheitszustandes und eine Optimierung der Behandlung für den Privatmann soll somit erleichtert und verbessert werden.

In der Praxis zeigt sich, dass einerseits besonders bei dem modernen Patienten der Wunsch einer autonomen Verwaltung seiner Analysedaten zum selbständigen und optimierten Handling der Krankheit besteht, andererseits jedoch eine Datenverarbeitung oftmals ungenutzt bleibt, da es sich bei den Benutzern der Analysegeräte häufig um ältere Personen handelt, die mit modernen Methoden der Datenverarbeitung nicht vertraut sind.

Es ist deshalb erstrebenswert dem Kunden zunächst nur die Möglichkeit der Datenverarbeitung anzubieten, ohne hierfür zusätzliche Kosten zu verursachen. In Hinblick auf eine weitere Kostenreduktion soll nicht nur eine externe Datenverarbeitung erfolgen, sondern auch bereits vorhandene im Analysegerät integrierte Gerätebausteine, die zur Datenverarbeitung dienen, aus dem Analysegerät ausgeschlossen werden. Ein solcher Gerätebaustein dient z. B. zur Datenübertragung vom Analysegerät zur externen Datenverarbeitungseinheit. Ein derartiges Datenübertragungsmodul wird dem Kunden als zusätzlicher Baustein gesondert angeboten und kann bei Bedarf an das Analysegerät angeschlossen werden. Dem Benutzer wird somit ermöglicht, selbst zu entscheiden, zusätzliche Kosten für eine eigene Datenverarbeitung zu leisten.

Die Bereitstellung einer gesonderten Datenübertragungseinheit als zusätzlicher Baustein eines Analysegerätes, setzt jedoch voraus, dass eine einfache Handhabung eines Anschlusses der Datenverarbeitungseinheit an das Analysegerät möglich ist. Weiterhin sollte ein derartiger Anschluss eines Datenübertragungsmoduls ebenfalls kostengünstig sein und keine wiederum aufwendigen baulichen Maßnahmen z. B. am Analysegerät benötigen, so dass das Angebot einer zusätzlichen Datenverarbeitung attraktiv bleibt. Im Stand der Technik wird ein kostengünstiger Anschluss eines Datenübertragungsmoduls offenbart, der sich als Schnittstelle für ein Datenübertragungsmodul eine in der Regel bereits vorhandene Kalibrationsmodul-Schnittstelle zu Nutzen macht.

Eine Kalibrationsmodul-Schnittstelle eines Analysesystems dient zur Ankopplung eines sogenannten Kalibrationsmoduls. Das Kalibrationsmodul beinhaltet eine Codierung, die auf einen Chip gespeichert ist und dessen Daten zur Kalibration von Messdaten eines Analysegerätes benutzt wird. Mit Hilfe einer solche Kalibration werden z. B. Chargenschwankungen im Reagenzsystem kompensiert.

Beispielhaft sind solche Systeme von der Firma Roche Diagnostics GmbH bekannt, die für die Messung von Blutzucker dienen (AccuChek Advantage Family®). Bei einem solchen Glucosemessgerät erfolgt die Glucosebestimmung mittels Teststreifen, auf die eine Probe aufgegeben wird. Die Probe reagiert mit einem Reagenzsystem des Teststreifens in der Weise, dass mittels des Analysegerätes der Glukosegehalt der Probe quantifiziert werden kann. Das oben genannte Kalibrationsmodul auch häufig Codekey genannt, wird z. B. einer jeweiligen Teststreifenpackung beigelegt, so dass chargenbedingte Schwankungen, die bei der Herstellung der Teststreifen entstehen, berücksichtigt werden können. Hierzu wird das Kalibrationsmodul an die vorgesehene Schnittstelle angeschlossen, und die auf einem Chip gespeicherte Codierung kann ausgelesen werden. Mittels der Codierung erfolgt im Analysegerät eine Korrektur der Messdaten.

Im Stand der Technik wird für einen Datentransfer zwischen dem Analysegerät und einer Datenverarbeitungseinheit das Kalibrationsmodul aus der Schnittstelle entnommen, so dass die Schnittstelle zum Datentransfer verwendet werden kann. Hierfür wird ein Datentransferkabel in die Schnittstelle eingesetzt. Die zuvor gemessenen und gespeicherten Daten werden über die Schnittstelle mittels des Datentransferkabels an die Datenverarbeitungseinheit weitergeleitet, indem z. B. eine Verbindung des Datentransferkabels mit einem PC hergestellt wird. Eine Datenübertragung kann dann z. B. über die PC- Software CAMIT® von Roche Diagnostics GmbH erfolgen.

Geeignete Kabel, die mit einer Kalibrationsmodul-Schnittstelle kommunizieren können, werden z. B. von der Firma Roche Diagnostics GmbH unter den Namen "AccuChek Interface Kabel®" oder "Professional Cable®" angeboten.

Die Dokumente WO 00/45696, DE19703854 und DE4305058 offenbaren Datenübertragungsmodule gemäß dem Stand der Technik.

Nachteile des Standes der Technik sind jedoch, dass bei der Datenübertragung ein elektrischer Kontakt zwischen dem Analysegerät und der Netzspannung der Datenverarbeitungseinheit hergestellt wird. Über die Kontaktkette: Netzspannung - PC - Analysegerät - Teststreifen - Blutstropfen - Benutzer, besteht für den Kunden das potentielle Risiko mit der Netzspannung in Kontakt zu kommen.

Bei der Verwendung derartiger Analysegeräte wird deshalb häufig über ein Manual darauf hingewiesen, dass mittels des Datentransferkabels, das mit der Datenverarbeitungseinheit verbunden ist, ein Risiko für den Benutzer besteht, sich zu elektrisieren, was besondere Vorsichtmaßnahmen bei der Bedienung erfordert.

Der Erfindung liegt die Aufgabe zugrunde, ein Analysesystem mit einem steckbaren Datenübertragungsmodul zu konzipieren, mit dem eine bequeme, kostengünstige und sichere Auswertung von Analysedaten möglich ist. Insbesondere soll ein elektrischer Kontakt zu der Datenverarbeitungseinheit bei der Übertragung von Daten vermieden werden.

Die Erfindung betrifft ein steckbares Datenübertragungsmodul, welches eine Schnittstelle beinhaltet, die mit einer Kalibrationsmodul-Schnittstelle eines Analysegerätes kompatibel ist und einen Datentransfer von elektrischen Signalen zwischen dem Datenübertragungsmodul und dem Analysegerät ermöglicht. Das Datenübertragungsmodul beinhaltet weiterhin eine Umwandlungseinheit, die elektrische Signale in elektromagnetische oder akustische Signale oder elektromagnetische oder akustische Signale in elektrische Signale umwandelt sowie eine Kommunikationseinheit, die einen Datentransfer der elektromagnetischen oder akustischen Signale zwischen dem Datenübertragungsmodul und einer Datenverarbeitungseinheit ermöglicht.

Elektromagnetische Signale im Sinne der Erfindung sind z. B. optische Signale.

Ein weiterer Aspekt der Erfindung ist ein Analysesystem mit einem steckbaren Datenübertragungsmodul, welches Daten von einem Analysegerät zu einer Datenverarbeitungseinheit überträgt beinhaltend ein Analysegerät mit einer Schnittstelle, an die ein Kalibrationsmodul oder alternativ ein steckbares Datenübertragungsmodul angesteckt werden kann sowie ein steckbares Datenübertragungsmodul. Das Datenübertragungsmodul weist eine Schnittstelle auf, die einen Datentransfer von elektrischen Signalen zwischen dem Datenübertragungsmodul und der Schnittstelle des Analysegerätes ermöglicht. Mit Hilfe einer Umwandlungseinheit werden die elektrischen Signale in elektromagnetische, oder akustische Signale oder elektromagnetische oder akustische Signale in elektrische Signale umgewandelt.

Eine Umwandlungseinheit im Sinne der Erfindung kann folglich je nach Bedarf sowohl elektrische Signale in akustische und elektromagnetische Signale umwandeln. Es ist jedoch auch denkbar, daß eine Umwandlung von elektrischen Signalen in ausschließlich akustische oder elektromagnetische Signale hinreichend ist, so daß das Modul nur für eine Art der Signalumwandlung geeignet ist. Unabhängig von der Art der Signalumwandlung, beinhaltet eine Signalumwandlung in der Regel die Möglichkeit, elektrische Signale in nicht-elektrische Signale umzuwandeln sowie den komplementären Weg, nicht-elektrische Signale in elektrische Signale zu transferieren. Eine vereinfachte Ausführungsform, die nur einen Weg der Signalumwandlung ermöglicht, und den komplementären Weg nicht zuläßt, ist natürlich und erdenklich in Abhängigkeit des jeweiligen Anwendungsgebietes.

Mittels einer Kommunikationseinheit ist ein Datentransfer der elektromagnetischen oder akustischen Daten zwischen dem Datenübertragungsmodul und einer Datenverarbeitungseinheit möglich.

Die Erfindung ermöglicht somit für den Benutzer über einen steckbaren Kontakt eine einfache Handhabung, die einen schnellen Anschluss des Datenübertragungsmoduls an ein Analysegerät gewährleistet. Die Daten des Analysegerätes können auf diese Weise über die Schnittstelle mit dem Datenübertragungsmodul ausgetauscht werden. Mittels des Datenübertragungsmoduls findet eine Umwandlung der Signale statt. Dies ermöglicht einen Datenaustausch zwischen Datenübertragungsmodul und Datenverarbeitungseinheit, die eine galvanisch entkoppelte Datenübertragung gewährleistet. Für den Benutzer besteht kein Risiko z. B. mit der Netzspannung eines PCs in Kontakt zu kommen. Weitere Vorteile bei der Handhabung ergeben sich dadurch, dass eine Datenübertragung durch den Raum erfolgt und keine Verbindung, wie z. B. durch ein Kabel, zwischen Datenübertragungsmodul und Datenverarbeitungseinheit notwendig ist.

Im Datenübertragungsmodul kann z. B. ein Infrarotmodul integriert sein, das zum Senden und Empfangen von IR-Strahlung geeignet ist, so dass z. B. mittels eines IR-Senders Daten an die Datenverarbeitungseinheit weitergeleitet werden. Das Datenübertragungsmodul ist dann in der Lage elektrische Signale in IR-Signale oder IR-Signale in elektrische Signale umzuwandeln. Die Datenverarbeitungseinheit registriert z. B. über einen IR-Empfänger die Signale. Das IR-Modul kann vorteilhaft so ausgestaltet sein, dass die Signale durch handelsübliche IR-Empfangs/Sendeeinheiten, wie sie z. B. an PCs verwendet werden, registriert werden können. Eine Übertragung der Daten ist somit bei handelsüblicher Sender/Empfangsleistung über einen räumlichen Abstand von ca. 1 m möglich.

Unter den genannten Bedingungen erweist sich die Verwendung eines Mikrocontrollers als besonders geeignet, der Platz- Kosten- und Funktionsvorteile für das Datenübertragungsmodul bietet, so dass z. B. notwendige Schaltkreise, Impulsmodulation und automatische Spannungsabschaltung in einem Baustein integriert sind. Dies gewährleistet z. B., dass eine Datenübertragung erst dann stattfindet, wenn das Analysegerät funktionsbereit ist, so dass eine fehlerhafte Datenübertragung vermieden werden kann.

Es ist jedoch auch die Verwendung eines Funkmoduls denkbar, das zum Senden und Empfangen von RF-Signalen geeignet ist. Hierbei werden elektrische Signale in RF-Signale oder RF-Signale in elektrische Signale umgesetzt.

Wenn eine Beschränkung auf eine bestimmte Signalumwandlung nicht erwünscht ist, um eine größere Kompatibilität mit Datenverarbeitungseinheiten zu erreichen, ist natürlich auch eine beliebige Kombination von signalspezifischen Modulen bzw. Signalumwandlungen möglich. In diesem Fall würde ein Datenübertragungsmodul beispielsweise ein IR- und ein RF-Modul aufweisen.

Bei einer bevorzugten Ausführungsform dient das Analysegerät, wie bereits beispielhaft beschrieben, zur Glucosekonzentrationsmessung, die anhand von Testelementen bestimmt wird. Da besonders bei Diabetes oftmals eine Glucosemessung mehrmals am Tag erfolgt, werden Analysegeräte zur Glucosemessung im Speziellen für den privaten Gebrauch hergestellt. Diabetes gehört im besonderen Maße zu den Krankheiten, bei denen eine gute Überwachung des Krankheitsbildes hilfreich ist, um Schädigungen wie z. B. Erblindung zu vermeiden. Eine Überwachung der Krankheit mit Hilfe einer geeigneten Datenverwaltung, die von dem Patienten bei Bedarf selber durchgeführt werden kann, ist hier also besonders gefragt.

Im Rahmen der Erfindung ist natürlich jegliche Art von Analysengeräten denkbar, wie z. B. Koagulationsmessgeräte.

In einer weiteren bevorzugten Ausführungsform beinhaltet das Analysesystem ein Kalibrationsmodul, das an die Schnittstelle des Analysegerätes angesteckt wird. Ein solches Kalibrationsmodul (Codekey) wird in der Regel z. B. beim Kauf von Testelementen der Packung beigefügt. Der Kunde wird dann dazu angehalten, das Kalibrationsmodul über die Schnittstelle mit dem Analysegerät zu verbinden, so dass z. B. eine Korrektur von Messdaten erfolgt. Die Tatsache, dass die identische Schnittstelle des Analysegerätes ebenfalls zur Verbindung des Gerätes mit dem Datenübertragungsmodul geeignet ist, vermeidet zusätzliche Maßnahmen beim Bau eines Analysengerätes. Der Kunde kann sich also jederzeit für eine Datenverarbeitung entscheiden, auch wenn er dies beim Kauf eines Analysegerätes ehemals nicht bedacht hat, so dass hierfür nur eine nachträgliche Anschaffung eines erfindungsgemäßen Datenübertragungsmoduls notwendig ist.

Das Datenübertragungsmodul bezieht seine Energie vorteilhafterweise aus dem Analysesystem, beispielsweise kann jedoch auch eine Energieversorgungseinheit im Datenübertragungsmoduls integriert sein.

Ist im Datenübertragungsmodul eine Funktechnologie integriert, besteht neben den genannten Energieversorgungen noch die Möglichkeit, die Energie aus dem Geräteumfeld in Form einer Erregerschwingung zu beziehen, so dass das Datenübertragungsmodul Bestandteil eines Transponiersystems ist. Bei der Transpondertechnik kann das Modul seine Energie passiv aus dem Geräteumfeld beziehen, indem die Sendeantenne des Lesesystems den Transponder über ein magnetisches Wechselfeld kontinuierlich mit Energie versorgt. Transponder sind völlig wartungsfrei und haben eine hohe Lebensdauer. Bevorzugt kann im niederfrequenten Bereich (124 kHz bzw. 62 kHz) gearbeitet werden, so dass das Datenübertragungsmodul keinen besonderen Sicherheitsbestimmungen unterliegt.

Die Anwendung eines Transponders hat weiter den Vorteil, dass Daten auf einen Mikrochip geschrieben werden können, so dass diese Anwendung eine reversible Speicherung von Daten erlaubt. Die Vorteile der Datenspeicherung werden im weiteren Verlauf noch näher ausgeführt.

Generell bestehen, wie beschrieben, verschiedene Möglichkeit für ein Datenübertragungsmodul seine Energie zu beziehen. Da es sich bei den angeführten Analysegeräten im medizinischen Bereich oftmals um batteriebetriebene Geräte handelt, ist ein geringer Energieverbrauch erstrebenswert. Dieser kann z. B. durch einen möglichst kurzen Gebrauch des Datenübertragungsmoduls begünstigt werden. Bei einer bevorzugten Ausführung kann das Datenübertragungsmodul durch Kontakt mit der geräteseitigen Schnittstelle aktiviert werden oder durch einen Startimpuls der Datenverarbeitungseinheit oder des Analysesystems. Nach einer definierten Zeitspanne ohne Datentransfer wird das Datenübertragungsmodul in einer bevorzugten Ausführungsform deaktiviert. Dies gewährleistet, dass kein unnötiger Energieverbrauch durch das Datenübertragungsmodul stattfindet.

Die übertragenden Daten können mit Hilfe der Datenverarbeitungseinheit bearbeitet werden.

In einer weiteren, bevorzugten Ausführungsform beinhaltet das Datenübertragungsmodul eine Speichervorrichtung, die, wie beschrieben, z. B. durch ein Transpondersystem verwirklicht werden kann, so dass z. B. gespeicherte Daten auch ohne Kontakt des Datenübertragungsmoduls vom Analysesystem zur Datenverarbeitungseinheit transferiert werden können. Das Datenübertragungsmodul speichert bevorzugt automatisch die transferierten Daten und kann anschließend aus dem Analysegerät entfernt werden. Wegen seiner geringen Größe ist das Datenübertragungsmodul leicht zu transportieren. Der Benutzer ist somit in der Lage an einem ausgewählten Ort, die gespeicherten Daten auszulesen, ohne gezwungen zu sein das Analysegerät mit sich zu führen. Dies ist besonders für die Benutzer geeignet, die mit modernen Methoden der Datenverarbeitung nicht vertraut sind, jedoch eine Überwachung ihrer Daten wünschen. Die bevorzugte Ausführungsform der Erfindung ermöglicht folglich eine leicht zu handhabende Datenverarbeitung von den Personen, denen selbst keine Datenverarbeitung möglich ist und die somit eine Datenverarbeitung z. B. in der Praxis ihres behandelnden Arztes in Auftrag geben können. Der Benutzer ist auf diese Weise nicht darauf angewiesen, sich mit sonst notwendigen Schritten der Datenübertragung wie z. B. Übermittlung der Daten via Internet oder Speichern der Daten auf ein Speichermedium etc. auseinander zusetzen.

Ein weiterer Aspekt der Erfindung ist ein Verfahren zum Datentransfer zwischen einem Analysesystem und einer Datenverarbeitungseinheit.

Das Verfahren beinhaltet das Anstecken eines steckbaren Kalibrationsmoduls an eine Schnittstelle des Analysesystems und Herausziehen aus der Schnittstelle nach Übertragung von Daten des Kalibrationsmoduls an das Analysesystem, sowie das Anstecken eines steckbaren Datenübertragungsmoduls an die Schnittstelle des Analysesystems und Übermittlung von elektrischen Signalen des Analysesystems zum Datenübertragungsmoduls. Umwandlung der elektrischen Signale des Analysesystems in elektromagnetische oder akustische Signale mittels des Datenübertragungsmoduls und Transfer der umgewandelten Daten zu einer Datenverarbeitungseinheit.

Bei einer bevorzugten Ausführungsform des Verfahrens wird ein Datenübertragungsmodul oder ein Analysesystem wie beschrieben verwendet.

Eine bevorzugte Ausführungsform des Datenübertragungsmoduls ist für ein Verfahren wie beschrieben geeignet, sowie ein bevorzugtes Analysesystem ein Datenübertragungsmodul wie beschrieben beinhaltet.

Anhand der nachfolgenden Figuren werden einige Beispiele näher erläutert.
- FIG 1:: Analysegerät zur Messung von Glykosekonzentration - Vorder- und Hinteransicht
- FIG 2:: Analysegerät mit Codekey
- FIG 3:: Analysesystem mit Analysegerät und Datenübertragungsmodul
- FIG 4:: Datenübertragung zwischen Analysesystem und Datenverarbeitungseinheit

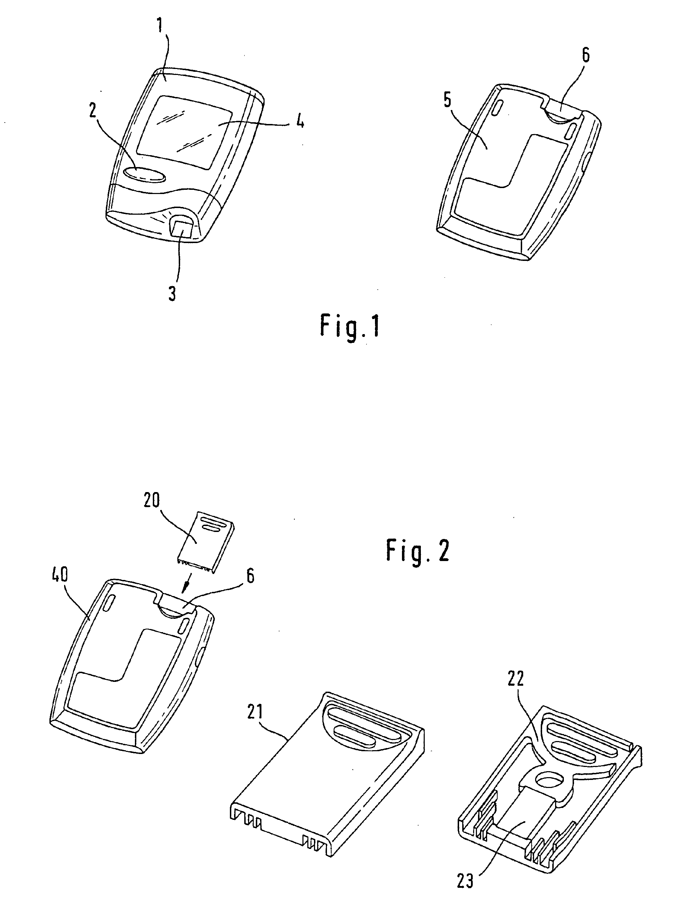

FIG 1 stellt die Vorderansicht (1) eines Glukosemessgerätes dar, das eine Bedienungstastatur (2) und ein Schacht für ein Teststreifen (3) aufweist. In dem Schacht (3) wird ein Teststreifen nach Probenaufgabe zur Vermessung des Glukosewertes in das Analysegerät eingeführt. Das Messergebnis wird mittels des Displays (4) dem Benutzer angezeigt. Das Analysegerät weist auf seiner Rückseite (5) einen weiteren Schacht (6) zum Einführen eines Kalibrationsmoduls auf. Dieses Kalibrationsmodul wird üblicherweise als Codekey bezeichnet. Ein solcher Codekey wird in der Regel einer Teststreifenpakkung beigelegt, so dass die Teststreifenchemie bei der Konzentrationsbestimmung des Glukosewertes mit berücksichtigen werden kann. Dieser Codekey enthält eine Codierung, die bei der Konzentrationsberechnung eine Korrektur der Messwerte ermöglicht und chargenspezifische Schwankung der Teststreifen mit einbezieht. Das Gerät ließt den gespeicherten Code aus und berücksichtigt diesen, so dass ein korrigiertes Messergebnis auf dem Bildschirm angezeigt wird. Erfindungsgemäß kann der Schacht (6) auch zur Verbindung des Analysegerätes mit einem Datenübertragungsmodul dienen. Nach Entfernung des Codekeys aus der Schnittstelle kann das Datenübertragungsmodul an die freie Schnittstelle gesteckt werden.

In FIG 2 wird beispielhaft ein Analysegerät (40) mit einem Codekey (20) gezeigt, der, wie es bereits im Stand der Technik bekannt ist, mit dem Analysegerät kontaktiert wird.

Der Codekey besitzt ein Kunststoffgehäuse (22), dessen Vorderansicht (21) ebenfalls in FIG 2 dargestellt ist. Das Kunststoffgehäuse ist so geschaffen, daß es mit dem Schacht (6) des Analysegerätes kompatibel ist. Die Daten des Codeykeys sind auf dem Chip (23) gespeichert und können nach dem Kontaktieren des Codekeys vom Analysesystem ausgelesen werden.

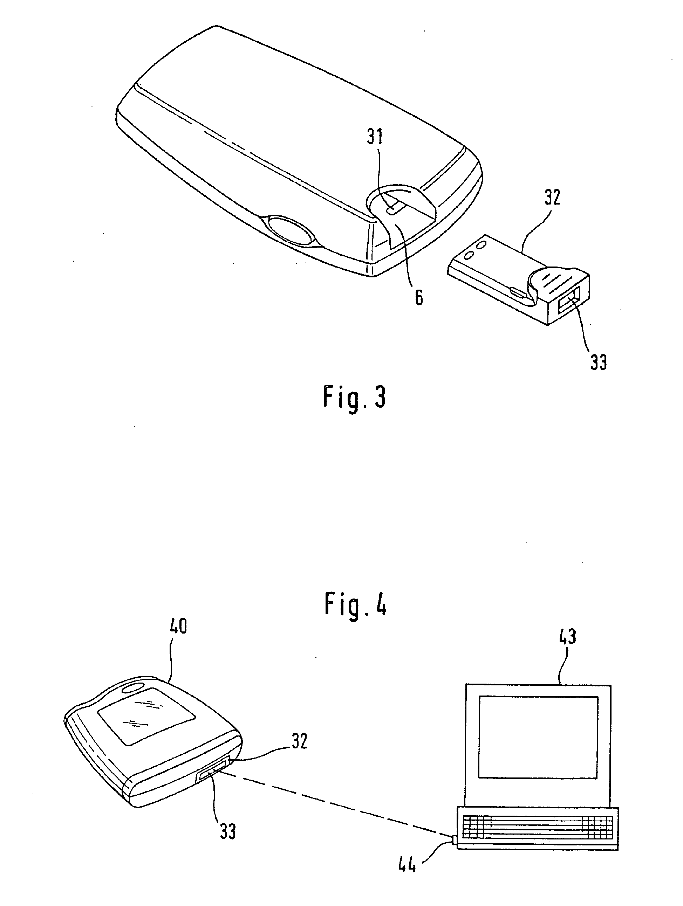

FIG 3 veranschaulicht detailliert den Schacht (6) und zeigt weiterhin beispielhaft ein Infrarot-Datenübertragungsmodul (32), das einen Infrarotsender/-empfänger (33) beinhaltet. Nach Einschieben des Datenübertragungsmoduls (32) wird das Analysegerät über die Kontakte (31) mit dem Datenübertragungsmodul kontaktiert. Durch die Kontaktierung des Datenübertragungsmoduls mit dem Analysegerät wird das Datenübertragungsmodul automatisch nach einer definierten Zeitspanne aktiviert. Über die Schnittstelle (31) werden elektronische Signale des Analysegerätes von zuvor gespeicherten Daten zu dem Datenübertragungsmodul transferiert. Das Infrarot-Datenübertragungsmodul ist nun in der Lage, die elektrischen Signale in Infrarot-Signale umzuwandeln.

Der Datentransfer zwischen Analysegerät und Datenverarbeitungseinheit wird in FIG 4 verdeutlicht. Sobald das Infrarot-Datenübertragungsmodul (32) in das Messgerät (40) eingeschoben wird, ist das Gerät empfangs- und sendebereit. Wahlweise können nun Signale von dem PC (43) an das Analysegerät (40) oder umgekehrt gesendet werden. Werden Daten von dem PC (43) auf das Analysegerät (40) übertragen, wird zunächst die geeignete Software auf den PC (43) aufgerufen. Der PC (43) sendet über sein Infrarot-Modul (44) Lichtimpulse im IR-Bereich aus. Mittels eines Infrarot-Empfängers (33) empfängt das Datenübertragungsmodul (32) die gesendeten Lichtimpulse und wandelt diese in elektrische Impulse um. Dabei wird beispielsweise ein Lichtimpuls dem Zahlenwert 0 zugeordnet und das Ausbleiben eines Lichtimpulses dem Zahlenwert 1. Die Daten werden auf dem messgerätinternen seriellen Bus gelegt. Mittels des Analysegerätes können die Daten zur weiteren Verarbeitung verwendet werden. So ist es beispielsweise möglich, eine Datenverarbeitung nicht nur mittels eines PCs bereitzustellen. Ebenso können auch aus der Datenverarbeitung gewonnene Daten, z. B. zur Kalibrierung neuer Messdaten im Analysesystem, verwendet werden.

Ein Analysesystem mit einem erfindungsgemäßen Datenübertragungsmodul bietet folglich vielfältige Anwendungsmöglichkeiten zur Verwaltung von Messdaten, die eine Optimierung z. B. der Analysen- oder Behandlungsmethoden zulässt. Aufgrund des kostengünstigen und einfach zu handhabenden Datenübertragungsmoduls wird dem Benutzer erleichtert, eine Datenverarbeitung der Messdaten zu nutzen.

## Patentansprüche

1. Steckbares Datenübertragungsmodul (32), beinhaltend
e ine Umwandlungseinheit, die elektrische Signale in elektromagnetische oder akustische Signale oder elektromagnetische oder akustische Signale in elektrische Signale umwandelt,
e ine Kommunikationseinheit (33), die einen Datentransfer der elektromagnetischen oder akustischen Signale zwischen dem Datenübertragungsmodul (32) und einer Datenverarbeitungseinheit (43) ermöglicht,
**dadurch gekennzeichnet, dass** es weiterhin aufweist
e ine Schnittstelle, die mit einer Kalibrationsmodulschnittstelle eines Analysegerätes (40) kompatibel ist und einen Datentransfer von elektrischen Signalen zwischen dem Datenübertragungsmodul (32) und dem Analysegerät (40) ermöglicht, wobei das steckbare Datenübertragungsmodul (32) in einen Schacht (6) des Analysegerätes (40) zum Einführen eines Kalibrationsmoduls einführbar ist.

2. Steckbares Datenübertragungsmodul (32) gemäß Anspruch 1,
bei dem im Datenübertragungsmodul (32) ein Infrarotmodul oder ein Funkmodul integriert ist.

3. Steckbares Datenübertragungsmodul (32) gemäß Anspruch 1,
bei dem eine Energieversorgungseinheit integriert ist.

4. Analysesystem mit einem steckbaren Datenübertragungsmodul (32), welches einen Datentransfer zwischen einem Analysegerät (40) und einer Datenverarbeitungseinheit (43) ermöglicht, **dadurch gekennzeichnet, dass** es enthält
e in Analysegerät (40) mit einem Schacht (6) zum Einführen eines Kalibrationsmoduls (20) und einer Schnittstelle, an die das Kalibrationsmodul (20) oder alternativ ein steckbares Datenübertragungsmodul (32) angesteckt werden kann, und
e in steckbares Datenübertragungsmodul (32) nach einem der Ansprüche 1 - 3.

5. Analysesystem gemäß Anspruch 4,
bei dem das Analysegerät (40) eine Auswerteinheit für Testelemente besitzt und bevorzugt ein Gerät zur Glucosekonzentrationsmessung ist.

6. Analysesystem gemäß Anspruch 4,
das ein Kalibrationsmodul beinhaltet, das an die Schnittstelle des Analysegerätes angesteckt wird.

7. Analysesystem gemäß Anspruch 4,
bei dem das Datenübertragungsmodul (32) seine Energie aus dem Analysesystem bezieht.

8. Analysesystem gemäß Anspruch 4,
bei dem das Datenübertragungsmodul (32) ein Funkmodul beinhaltet und Bestandteil eines Transpondersystems ist.

9. Verfahren zum Datentransfer zwischen einem Analysesystem und einer Datenverarbeitungseinheit (43) beinhaltend
- Anstecken eines steckbaren Kalibrationsmoduls (20) an eine Schnittstelle eines Analysegerätes (40), wobei das Kalibrationsmodul (20) in einen Schacht (6) des Analysegerätes (40) eingeführt wird, und Herausziehen aus der Schnittstelle nach Übertragung von Daten des Kalibrationsmoduls (20) an das Analysegerät (40),
- Anstecken eines steckbaren Datenübertragungsmoduls (32) an die Schnittstelle des Analysegerätes (40), wobei das Datenübertragungsmodul (32) in den Schacht (6) des Analysegerätes (40) eingeführt wird, und
- Übermittlung von elektrischen Signalen des Analysegerätes (40) zum Datenübertragungsmodul (32),
- Umwandlung der elektrischen Signale des Analysegerätes (40) in elektromagnetische oder akustische Signale mittels des Datenübertragungsmoduls (32),
- Transfer der umgewandelten Daten zu einer Datenverarbeitungseinheit (43).

10. Verfahren gemäß Anspruch 9,
bei dem das Datenübertragungsmodul (32) durch Kontakt mit der geräteseitigen Schnittstelle aktiviert wird .

11. Verfahren gemäß Anspruch 9,
bei dem das Datenübertragungsmodul (32) durch einen Startimpuls der Datenverarbeitungseinheit (43) oder des Analysegerätes (40) aktiviert wird.

12. Verfahren gemäß Anspruch 9,
bei dem das Datenübertragungsmodul (32) nach einer definierten Zeitspanne, in der kein Datentransfer erfolgt ist, inaktiv wird.

13. Verfahren gemäß Anspruch 9,
bei dem im Datenübertragungsmodul (32) elektrische Signale in Infrarot(IR)-Signale oder Infrarot(IR)-Signale in elektrische Signale umgewandelt werden.

14. Verfahren gemäß Anspruch 9,
bei dem im Datenübertragungsmodul (32) elektrische Signale in Radiofrequenz(RF)-Signale oder Radiofrequenz(RF)-Signale in elektrische Signale umgewandelt werden.

15. Verfahren gemäß Anspruch 9,
bei dem das Datenübertragungsmodul (32) Daten speichert und die gespeicherten Daten auch ohne Kontakt des Datenübertragungsmoduls (32) mit dem Analysegerät (40) zur Datenverarbeitungseinheit (43) transferiert werden können.

16. Verfahren gemäß Anspruch 9,
bei dem ein Analysesystem nach einem der Ansprüche 4 - 8 verwendet wird.

## Claims

1. Plug-in data transfer module (32), comprising
- a conversion unit, which converts electrical signals into electromagnetic or acoustic signals or converts electromagnetic or acoustic signals into electrical signals and
- a communication unit (33), which allows a data transfer of the electromagnetic or acoustic signals between the data transfer module (32) and a data processing unit (43),
**characterized in that** it also has
- an interface, which is compatible with a calibration module interface of an analytical instrument (40) and allows a data transfer of electrical signals between the data transfer module (32) and the analytical instrument (40), the plug-in data transfer module (32) being insertable into a shaft (6) of the analytical instrument (40) for the insertion of a calibration module.

2. Plug-in data transfer module (32) according to Claim 1,
in which an infrared module or a radio module is integrated in the data transfer module (32).

3. Plug-in data transfer module (32) according to Claim 1,
in which an energy supply unit is integrated.

4. Analysis system with a plug-in data transfer module (32), which allows a data transfer between an analytical instrument (40) and a data processing unit (43), **characterized in that** it includes
- an analytical instrument (40) with a shaft (6) for inserting a calibration module (20) and an interface, to which the calibration module (20) or alternatively a plug-in data transfer module (32) can be attached, and
- a plug-in data transfer module (32) according to one of Claims 1-3.

5. Analysis system according to Claim 4,
in which the analytical instrument (40) has an evaluation unit for test elements and is preferably an instrument for measuring glucose concentrations.

6. Analysis system according to Claim 4,
which comprises a calibration module which is attached to the interface of the analytical instrument.

7. Analysis system according to Claim 4,
in which the data transfer module (32) draws its energy from the analysis system.

8. Analysis system according to Claim 4,
in which the data transfer module (32) comprises a radio module and is a component part of a transponder system.

9. Method for transferring data between an analysis system and a data processing unit (43) comprising
- attaching a plug-in calibration module (20) to an interface of an analytical instrument (40), wherein the calibration module (20) is inserted into a shaft (6) of the analytical instrument (40), and withdrawing it from the interface after the transfer of data from the calibration module (20) to the analytical instrument (40),
- attaching a plug-in data transfer module (32) to the interface of the analytical instrument (40), the data transfer module (32) being inserted into the shaft (6) of the analytical instrument (40), and
- transmitting electrical signals from the analytical instrument (40) to the data transfer module (32),
- converting the electrical signals of the analytical instrument (40) into electromagnetic or acoustic signals by means of the data transfer module (32),
- transferring the converted data to a data processing unit (43).

10. Method according to Claim 9,
in which the data transfer module (32) is activated by contact with the interface on the instrument side.

11. Method according to Claim 9,
in which the data transfer module (32) is activated by a starting pulse from the data processing unit (43) or the analytical instrument (40).

12. Method according to Claim 9,
in which the data transfer module (32) is deactivated after a defined time period in which no data transfer has occurred.

13. Method according to Claim 9,
in which electrical signals are converted into infrared (IR) signals or infrared (IR) signals are converted into electrical signals in the data transfer module (32).

14. Method according to Claim 9,
in which electrical signals are converted into radio-frequency (RF) signals or radio-frequency (RF) signals are converted into electrical signals in the data transfer module (32).

15. Method according to Claim 9,
in which the data transfer module (32) stores data and the stored data can be transferred to the data processing unit (43) even if the data transfer module (32) is not in contact with the analytical instrument (40).

16. Method according to Claim 9,
in which an analysis system according to one of Claims 4-8 is used.

## Revendications

1. Module enfichable de transmission de données (32), contenant
- une unité de conversion qui convertit des signaux électriques en signaux électromagnétiques ou acoustiques, ou bien des signaux électromagnétiques ou acoustiques en signaux électriques,
- une unité de communication (33) qui permet un transfert de données des signaux électromagnétiques ou acoustiques entre le module de transmission de données (32) et une unité de traitement de données (43),
**caractérisé en ce qu'**il présente en outre
- une interface qui est compatible avec une interface de module d'étalonnage d'un appareil d'analyse (40) et permet un transfert de données de signaux électriques entre le module de transmission de données (32) et l'appareil d'analyse (40), dans lequel le module enfichable de transmission de données (32) peut être introduit dans une fente (6) de l'appareil d'analyse (40) servant à l'introduction d'un module d'étalonnage.

2. Module enfichable de transmission de données (32) selon la revendication 1, dans lequel un module à infrarouge ou un module radio est intégré dans le module de transmission de données (32).

3. Module enfichable de transmission de données (32) selon la revendication 1, dans lequel est intégrée une unité d'alimentation en énergie.

4. Système d'analyse comprenant un module enfichable de transmission de données (32) qui permet un transfert de données entre un appareil d'analyse (40) et une unité de traitement de données (43), **caractérisé en ce qu'**il contient
- un appareil d'analyse (40) comportant une fente (6) servant à l'introduction d'un module d'étalonnage (20), et une interface au niveau de laquelle peut être enfiché le module d'étalonnage (20) ou bien, en variante, un module enfichable de transmission de données (32), et
- un module enfichable de transmission de données (32) selon l'une quelconque des revendications 1 à 3.

5. Système d'analyse selon la revendication 4, dans lequel l'appareil d'analyse (40) comprend une unité d'évaluation pour des éléments de test et est, de préférence, un appareil servant à mesurer la concentration en glucose.

6. Système d'analyse selon la revendication 4, qui contient un module d'étalonnage qui est enfiché au niveau de l'interface de l'appareil d'analyse.

7. Système d'analyse selon la revendication 4, dans lequel le module de transmission de données (32) tire son énergie du système d'analyse.

8. Système d'analyse selon la revendication 4, dans lequel le module de transmission de données (32) contient un module radio et fait partie d'un système de transpondeur.

9. Procédé pour le transfert de données entre un système d'analyse et une unité de traitement de données (43), comprenant
- l'enfichage d'un module d'étalonnage enfichable (20) au niveau d'une interface d'un appareil d'analyse (40), dans lequel le module d'étalonnage (20) est introduit dans une fente (6) de l'appareil d'analyse (40), et le débranchement par rapport à l'interface après transmission de données du module d'étalonnage (20) à l'appareil d'analyse (40),
- l'enfichage d'un module enfichable de transmission de données (32) au niveau de l'interface de l'appareil d'analyse (40), dans lequel le module de transmission de données (32) est introduit dans la fente (6) de l'appareil d'analyse (40), et
- la transmission de signaux électriques de l'appareil d'analyse (40), au module de transmission de données (32),
- la conversion des signaux électriques de l'appareil d'analyse (40) en signaux électromagnétiques ou acoustiques au moyen du module de transmission de données (32),
- le transfert des données converties, à une unité de traitement de données (43).

10. Procédé selon la revendication 9, dans lequel le module de transmission de données (32) est activé par contact avec l'interface située côté appareil.

11. Procédé selon la revendication 9, dans lequel le module de transmission de données (32) est activé par une impulsion de départ de l'unité de traitement de données (43) ou de l'appareil d'analyse (40).

12. Procédé selon la revendication 9, dans lequel le module de transmission de données (32) est inactif après un laps de temps défini, au cours duquel il ne se produit aucun transfert de données.

13. Procédé selon la revendication 9, dans lequel des signaux électriques sont convertis, dans le module de transmission de données (32), en signaux infrarouges (IR), ou bien des signaux infrarouges (IR) convertis en signaux électriques.

14. Procédé selon la revendication 9, dans lequel des signaux électriques sont convertis, dans le module de transmission de données (32), en signaux radiofréquence (RF), ou bien des signaux radiofréquence (RF) convertis en signaux électriques.

15. Procédé selon la revendication 9, dans lequel le module de transmission de données (32) stocke des données, et les données stockées peuvent être transférées à l'unité de traitement de données (43), même sans contact du module de transmission de données (32) avec l'appareil d'analyse (40).

16. Procédé selon la revendication 9, dans lequel un système d'analyse est utilisé selon l'une quelconque des revendications 4 à 8.
